# DEMANDE DE BREVET EUROPEEN

(11) **EP 1 433 459 A1**
(43) Date de publication de la demande: **30.06.2004**
(21) Numéro de dépôt: 03293310.3
(22) Date de dépôt: 23.12.2003
(51) Int. Cl.: A61K 7/02, A61K 7/13

(54) **Procédé pour teinter ou colorer des matières kératiniques humaines avec effet d'éclaircissement au moyen d'une composition comprenant un composé fluorescent et un azurant optique et composition**

(30) Priorité: 24.12.2002 FR 0216669
(71) Demandeur: L'OREAL, 75008 Paris (FR)
(72) Inventeur: Gourlaouen, Luc, 92600 Asnières (FR); Pastore, Florent, 92500 Rueil Malmaison (FR)
(74) Mandataire: Wattremez, Catherine

(57) **Abrégé**

La présente invention a pour objet un procédé pour teinter ou colorer, avec un effet éclaircissant, des matières kératiniques humaines, par application d'une composition comprenant, dans un milieu cosmétiquement acceptable, au moins un composé fluorescent et au moins un azurant optique.

Elle a de même pour objet une composition comprenant, dans un milieu cosmétiquement acceptable, au moins un composé fluorescent et au moins un azurant optique, solubles dans ledit milieu.

Elle concerne enfin un dispositif à plusieurs compartiments, dont l'un d'eux renferme une composition comprenant au moins un composé fluorescent et au moins un azurant optique et éventuellement au moins un colorant direct additionnel et/ou au moins une base d'oxydation et/ou au moins un coupleur, dans un milieu cosmétiquement acceptable, et l'autre compartiment renferme une composition comprenant au moins un agent oxydant.

## Description

La présente invention a pour objet un procédé pour teinter ou colorer des matières kératiniques humaines, par application d'une composition comprenant, dans un milieu cosmétiquement acceptable, au moins un composé fluorescent et au moins un azurant optique. Elle a de même pour objet une composition comprenant, dans un milieu cosmétiquement acceptable, au moins un composé fluorescent et au moins un azurant optique, solubles dans ledit milieu, ainsi qu'un dispositif à plusieurs compartiments dont l'un d'eux comprend au moins un composé fluorescent et au moins un azurant optique, et l'autre au moins un agent oxydant.

Il est fréquent que les personnes ayant une peau colorée, voire foncée, désirent s'éclaircir la peau et utilisent dans ce but des compositions cosmétiques ou dermatologiques contenant des agents de blanchiment.
Les substances les plus utilisées comme agent de blanchiment sont l'hydroquinone et ses dérivés, l'acide kojique et ses dérivés, l'acide azélaïque, l'arbutine et ses dérivés, seuls ou en association avec d'autres actifs.
Toutefois ces agents ne sont pas dépourvus d'inconvénients. En particulier, il est nécessaire de les utiliser de façon prolongée et en des quantités élevées, pour obtenir un effet de blanchiment de la peau. De plus, on n'observe pas un effet immédiat à l'application de compositions les comprenant.
Quant à l'hydroquinone et ses dérivés, ils sont connus pour leur cytotoxicité vis-à-vis du mélanocyte.
Enfin, l'acide kojique et ses dérivés présentent l'inconvénient d'être coûteux et de ne pouvoir, pour cette raison, être utilisés en quantité importante dans des produits à large diffusion commerciale.

Il subsiste donc le besoin de compositions cosmétiques permettant d'obtenir un teint plus clair, uniforme, homogène, d'aspect naturel.

Dans le domaine capillaire, pour obtenir une coloration plus claire, on met classiquement en oeuvre un procédé de décoloration chimique. Ce procédé consiste à décolorer les mélanines de ces fibres par un système oxydant, généralement constitué par du peroxyde d'hydrogène associé ou non à des persels. Cette opération peut ou non être réalisée en présence de colorants directs et/ou de colorants d'oxydation.
Ce système de décoloration présente l'inconvénient de dégrader les fibres et d'altérer leurs propriétés cosmétiques. Les cheveux ont en effet tendance à devenir rêches, plus difficilement démêlables et plus fragiles.

Il est donc souhaitable de pouvoir disposer de compositions qui permettent d'éclaircir tout en colorant les cheveux et autres fibres kératiniques humaines, de manière esthétique, sans dégrader ces fibres.

La présente invention a donc pour objet de proposer un procédé permettant de teinter ou de colorer des matières kératiniques humaines et une composition ne posant pas les difficultés mentionnées ci-dessus.

Ainsi, un premier objet de l'invention est constitué par un procédé pour teinter ou colorer avec effet éclaircissant des matières kératiniques humaines, consistant à appliquer sur lesdites matières une composition comprenant, dans un milieu cosmétiquement acceptable, au moins un composé fluorescent et au moins un azurant optique.

Un deuxième objet de l'invention concerne une composition comprenant, dans un milieu cosmétiquement acceptable, au moins un composé fluorescent soluble dans le milieu et au moins un azurant optique soluble dans le milieu.

Un troisième objet de l'invention est constitué par un dispositif à plusieurs compartiments, comprenant au moins un compartiment renfermant une composition comprenant au moins un composé fluorescent et au moins un azurant optique et éventuellement au moins un colorant direct additionnel et/ou au moins une base d'oxydation et/ou au moins un coupleur, dans un milieu cosmétiquement acceptable, et au moins un autre compartiment renfermant une composition renfermant au moins un agent oxydant.

La présente invention permet de teinter ou colorer tout en éclaircissant, des matières kératiniques humaines, sans altération de ces dernières.

Plus particulièrement, le procédé selon l'invention permet d'obtenir une coloration ou une teinte pour laquelle la réflectance des matières traitées conformément à l'invention, mesurée entre 550 et 700 nm, est supérieure à la réflectance des matières non traitées.

L'invention permet d'obtenir une coloration ou teinte plus claire que la coloration ou teinte naturelle, avec un effet esthétique très satisfaisant.

On a de plus constaté l'existence d'une interaction entre le composé fluorescent et l'azurant optique dans la composition augmentant le phénomène d'éclaircissement et élargissant le domaine dans lequel il est perceptible à l'oeil.
En effet, lors d'une application sur une peau dont la luminance est plus particulièrement inférieure ou égale à 55, ou sur des fibres kératiniques dont la hauteur de ton est avantageusement inférieure ou égale à 6, la réflectance de la matière traitée avec l'association des deux composés est supérieure à celle d'une matière traitée avec un composé fluorescent seul ; la réflectance d'une matière non traitée, entre 550 et 700 nm, étant inférieure à celle des matières traitées d'une part avec le composé fluorescent et d'autre part, avec son association avec un azurant optique.
Dans le cas d'une composition ne comprenant que l'azurant optique, la réflectance d'une matière kératinique traitée est voisine de celle d'une matière non traitée.
Par ailleurs, le domaine dans lequel la réflectance des matières traitées est supérieure à celle des matières non traitées est élargi dans le cas d'une composition associant les deux composés par rapport à celle d'une composition ne comprenant que le composé fluorescent.

Enfin, dans le cas de compositions appliquées sur des fibres kératiniques, comme notamment les cheveux, les composés mis en oeuvre présentent une bonne affinité tinctoriale pour ces fibres, de bonnes propriétés de ténacité vis-à-vis des agents extérieurs.

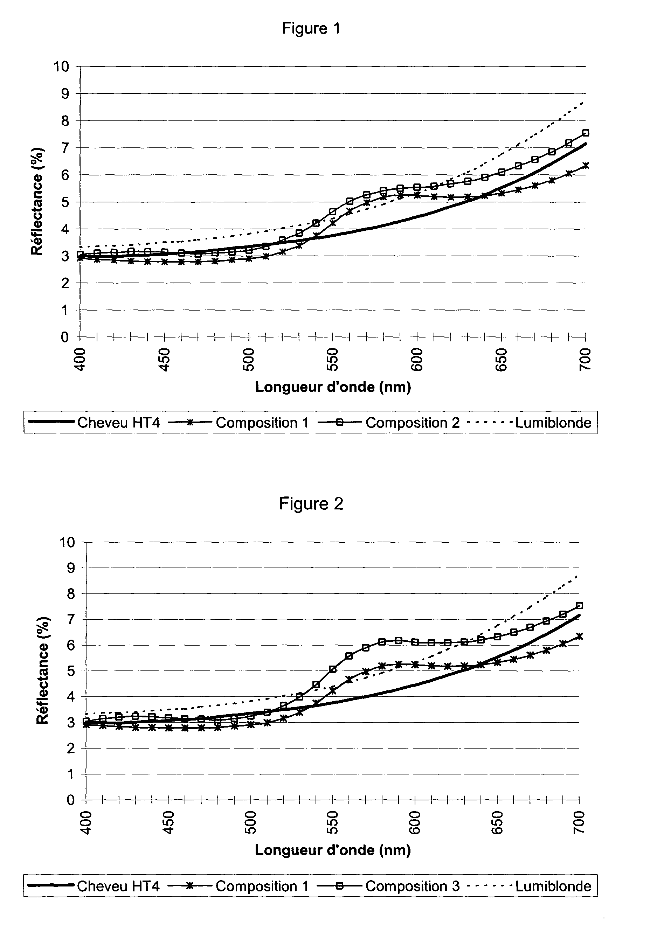

Mais d'autres caractéristiques et avantages de la présente invention apparaîtront plus clairement à la lecture de la description et des exemples qui vont suivre, ainsi que des figures 1 et 2 annexées qui représentent des courbes de réflectance en fonction de la longueur d'onde, de cheveux châtains non traités, de cheveux traités avec un produit commercial ; avec une composition comprenant un composé fluorescent seul et avec une composition comprenant l'association d'un composé fluorescent et d'un azurant optique.

Tout d'abord, les matières kératiniques traitées conformément au procédé selon l'invention sont d'origine humaine. Dans ce qui va suivre, il sera fait mention de matières kératiniques sachant qu'il s'agit de matières kératiniques humaines.
En outre, elles peuvent se trouver sous la forme de fibres ou non. Ainsi, lesdites matières kératiniques peuvent être la peau, les cheveux, les cils, les sourcils, la barbe, la moustache.

Selon un premier mode de réalisation de l'invention, la matière kératinique traitée est la peau. Plus particulièrement, celle-ci présente une luminance L* dans le système C.I.E. L*a*b*, mesurée au moyen d'un Colorimètre Minolta CM 2002, inférieure ou égale à 55, Il est rappelé qu'une valeur de 0 pour L* équivaut au noir et 100 au blanc.
Les types de peau correspondant à cette luminance sont la peau asiatique, la peau africaine, la peau afro-américaine, la peau hispano-américaine, la peau indienne et la peau maghrébine.

Selon un deuxième mode de réalisation de l'invention, les matières kératiniques traitées se trouvent sous la forme de fibres, et plus particulièrement de fibres kératiniques pigmentées ou de fibres colorées artificiellement. De préférence, ces fibres sont des cheveux.
Avantageusement, les cheveux pigmentés ou colorés artificiellement présentent une hauteur de ton inférieure ou égale à 6 (blond foncé) et de préférence inférieure ou égale à 4 (châtain).
Rappelons que la notion de "ton" repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Sciences des traitements capillaires » de Charles ZVIAK 1988, Ed.Masson, pp.215 et 278.

Ainsi que cela a été précisé auparavant, le procédé selon l'invention consiste donc à appliquer sur une matière kératinique, une composition comprenant, dans un milieu cosmétiquement acceptable, au moins un composé fluorescent et au moins un azurant optique, afin de teinter ou colorer avec un effet éclaircissant cette matière kératinique.

Pour plus de clarté, la composition mise en oeuvre dans le procédé selon l'invention va tout d'abord être détaillée.

Le composé fluorescent entrant dans la composition mise en oeuvre dans le procédé selon l'invention, est plus particulièrement choisi parmi les composés qui absorbent la lumière dans la partie visible du spectre et éventuellement dans la zone de l'ultraviolet, et ré-émettent une lumière fluorescente dans le spectre du visible, de plus grande longueur d'onde que celle de la lumière absorbée. De manière appropriée, la longueur d'onde de la lumière ré-émise est comprise entre 500 et 700 nm.

Conformément au procédé de l'invention, le ou les composés fluorescents peuvent se trouver sous une forme soluble ou non dans le milieu de la composition, à température ambiante (entre 15 et 25°C).

Dans ce procédé, de préférence, le composé fluorescent est choisi parmi les composés solubles dans le milieu de la composition.

Selon un mode de réalisation particulier du procédé de l'invention, le composé fluorescent est soluble dans le milieu de la composition à au moins 1 g/l et de préférence à au moins 5 g/l, à une température comprise entre 15 et 25°C.

A titre d'exemples de colorants fluorescents solubles dans le milieu susceptibles d'être mis en oeuvre, on peut citer les colorants fluorescents appartenant aux dérivés des familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges.

Parmi les composés fluorescents solubles dans le milieu susceptibles d'être mis en oeuvre dans le procédé selon l'invention, on peut citer plus particulièrement, sans toutefois vouloir s'y limiter :
- le Photosensitiving Dye NK-557 commercialisé par la société UBICHEM qui présente la structure suivante : 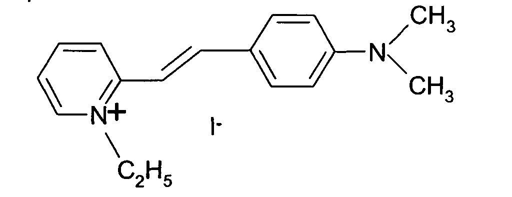 iodure de 2-[2-(4-diméthylamino)phényl éthényl]-1 éthyl-pyridinium ;
- le Jaune Brilliant B6GL commercialisé par la société SANDOZ et de structure suivante : 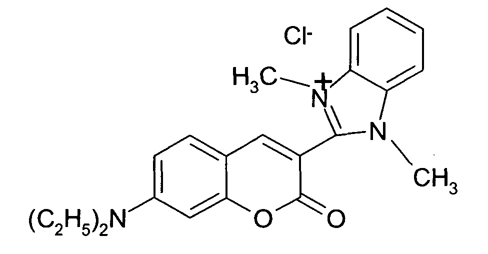
- le Basic Yellow 2, ou Auramine O commercialisé par les sociétés PROLABO, ALDRICH ou CARLO ERBA et de structure suivante : 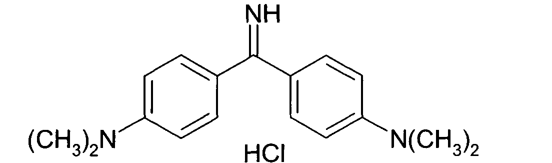
monochlorhydrate de 4,4'-(imidocarbonyl)bis(N,N-diméthylaniline) ; numéro CAS 2465-27-2.

En ce qui concerne les composés fluorescents non solubles dans le milieu pouvant être utilisés dans le procédé selon l'invention, on peut citer les composés inorganiques comme par exemple ceux à base d'oxyde de zinc, de sulfure de zinc. Parmi les composés fluorescents organiques non solubles dans le milieu, on peut citer les pigments fabriqués à partir de colorants fluorescents qui sont préalablement dissous dans une résine support afin d'obtenir une solution solide laquelle est ensuite broyée en une poudre de particules de résine présentant des propriétés fluorescentes. La préparation de tels pigments fluorescents est notamment décrite dans US 2851424 et US 3856550.

Les composés fluorescents insolubles peuvent ainsi être choisis parmi les résines colorées de polyamide et/ou de formaldéhyde / benzoguanamine et/ou de mélamine / formaldéhyde / sulfonamide, parmi les co-condensats aminotriazine /formaldéhyde / sulfonamide colorés et/ou parmi les paillettes polyester métallisées et/ou leurs mélanges. Ces composés fluorescents peuvent aussi se présenter sous la forme de dispersions aqueuses.

La composition peut comprendre un ou plusieurs composés fluorescents.

L'azurant optique entrant dans la composition mise en oeuvre dans l'invention, est choisi parmi les composés qui absorbent la lumière dans la partie ultra-violette du spectre, essentiellement dans l'UVA, à une longueur d'onde comprise entre 300 et 390 nm. Ces composés ré-émettent une lumière fluorescente dans le spectre du visible, comprise entre 400 et 525 nm.

Parmi les azurants optiques, conviennent tout spécialement les dérivés du stilbène, les dérivés coumariniques, les dérivés oxazole et benzoxazole, les dérivés imidazole.
A titre d'exemples, on peut citer notamment :
- le dérivé stilbénique de naphto-triazole (Tinopal GS de Ciba), le di-styryl-4,4' biphényle sulfonate di-sodique (nom CTFA : disodium distyrylbiphenyl disulfonate ; Tinopal CBS-X de Ciba = 4,4'-bis[(4,6-diamilino-1,3,5-triazin-2-yl)amino]stilbène-2,2'-disulfonate de sodium), le dérivé cationique d'aminocoumarine (Tinopal SWN Conc. de Ciba), le diéthylaminométhyl coumarine, le 4-méthyl 7-diéthyl coumarine, le 4,4'-bis[(4,6-dianilino-1,3,5-triazin-2-yl)amino]stilbène-2,2'-disulfonate de sodium (Tinopal SOP de Ciba), le 4,4'-bis-[(4-anilino-6-bis(2-hydroxyéthyl)amino-1,3,5-triazin-2-yl)amino]stilbène-2,2'-disulfonique acide (Tinopal UNPA-GX de Ciba), le 4,4'-bis-[anilino-6-morpholine-1,3,5-triazin-2-yl)amino] stilbène (Tinopal AMS-GX de Ciba), le 4,4'-bis-[(4-anilino-6-(2-hydroxy éthyl) méthylamino-1,3,5-triazin-2-yl)amino]stilbène-2,2'-disodium sulfonate (Tinopal 5BM-GX de Ciba),
- le 2,5 thiophène di-yl bis(5 ter-butyl-1,3 benzoxazole) (Uvitex OB de Ciba),
- le dérivé anionique du di-aminostilbène (dispersion dans l'eau, Leucophor BSB liquide de Clariant,
- les laques d'azurant optique (gamme Covazur de Wackherr).

Les azurants optiques utilisables dans la présente invention peuvent aussi se présenter sous la forme de copolymères, par exemple d'acrylates et/ou de méthacrylates, greffés par des groupement azurants optiques comme décrits dans la demande FR99-10942.

Là encore, la composition peut comprendre un ou plusieurs azurants optiques.

Selon un mode de réalisation très avantageux de l'invention, l'azurant optique est choisi de telle sorte que la longueur d'onde de la lumière que ré-émet au moins un azurant optique corresponde à la longueur d'onde d'absorption d'au moins un composé fluorescent présent.

Les azurants optiques préférentiellement utilisés selon l'invention sont le diéthylaminométhyl coumarine, le 4-méthyl 7-diéthyl coumarine, le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le di-styryl-4,4' biphényle sulfonate di-sodique et/ou leurs mélanges.

Selon un mode de réalisation particulier du procédé de l'invention, l'azurant optique est choisi parmi les composés qui sont solubles dans le milieu de la composition à au moins 0,1 g/l et de préférence à au moins 0,5 g/l, à une température comprise entre 15 et 25°C.

Les quantités minimales de composé(s) fluorescent(s) et d'azurant(s) optique(s) dans la composition, sont de préférence telles que la réflectance de la matière traitée, mesurée entre 550 et 700 nm, soit supérieure à la réflectance de la matière kératinique non traitée.

Plus particulièrement, la teneur en composé fluorescent représente 0,05 à 20% en poids par rapport au poids total de la composition, de préférence 0,05 à 10 % en poids par rapport à la même référence, de manière très avantageuse 0,1 à 5% en poids par rapport à la même référence.

Quant à la teneur en azurant optique, celle-ci représente plus particulièrement 0,05 à 10% en poids par rapport au poids total de la composition, de préférence 0,1 à 5% en poids par rapport à la même référence.

Le milieu cosmétiquement acceptable est généralement constitué par de l'eau ou par un mélange d'eau et d'au moins un solvant organique.
A titre de solvant organique, on peut par exemple citer, les alcanols, linéaires ou ramifiés, comprenant 1 à 4 atomes de carbone, tels que l'éthanol, l'isopropanol ; les polyols et éthers de polyols comme le glycérol, le 2-butoxyéthanol, le propylèneglycol, le monométhyléther de propylèneglycol, le monoéthyléther, le monométhyléther du diéthylèneglycol, le diméthoxyéthane, les alcools aromatiques comme l'alcool benzylique ou le phénoxyéthanol, les cétones comprenant 3 à 4 atomes de carbone, les acétates d'alkyle en C₁-C₄, ces composés étant seuls ou en mélanges.

A titre d'illustration, les solvants, s'ils sont présents, représentent 1 à 40 % en poids environ par rapport au poids total de la composition, et de manière plus avantageuse de 5 à 30 % en poids environ par rapport à la même référence.

Le pH de la composition mise en oeuvre dans le procédé selon l'invention est généralement compris entre 3 et 12 environ, et de préférence entre 5 et 11 environ.

Il peut être ajusté à la valeur désirée au moyen d'agents acidifiants ou alcalinisants habituellement utilisés.
Parmi les agents acidifiants, on peut citer, à titre d'exemples, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.
Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (I) suivante : 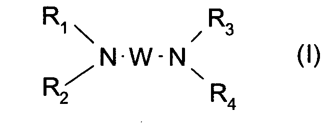 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ éventuellement porteur d'au moins un radical hydroxyle.

Selon une première variante du procédé selon l'invention, ce dernier consiste à appliquer sur des fibres kératiniques, comme les cheveux, pour les colorer tout en les éclaircissant, une composition comprenant au moins un composé fluorescent et au moins un azurant optique. Dans ce cas particulier, la composition appliquée comprend au moins un composé fluorescent et au moins un azurant optique qui se trouvent de préférence sous une forme soluble dans le milieu de la composition.

Selon cette première variante, la composition mise en oeuvre dans le procédé peut comprendre, outre le composé fluorescent et l'azurant optique, au moins un colorant direct additionnel.

Plus particulièrement, ledit colorant additionnel est de nature non ionique, cationique ou anionique.

Il peut par exemple être choisi parmi les colorants benzéniques nitrés rouges ou orangés suivants :
- le 1-hydroxy-3-nitro-4-N-(y-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine, et
- le1-amino-3-nitro-6-hydroxybenzène.
- le 1-(β-aminoéthyl)amino-2-nitro-4-(β-hydroxyéthyloxy) benzène,
- le 1-(β,γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine, et
- le 1-amino-2-nitro-4-hydroxy-5-méthylbenzène.

La composition mise en oeuvre dans le cadre de cette première variante peut également comprendre, en addition ou en remplacement de ces colorants benzéniques nitrés, un ou plusieurs colorants directs additionnels choisis parmi les colorants benzéniques nitrés jaunes, jaune-verts, bleus ou violets, les colorants azoïques, les colorants anthraquinoniques, naphtoquinoniques ou benzoquinoniques, les colorants indigoïdes, ou les colorants dérivés du triarylméthane.

Ces colorants directs additionnels peuvent notamment être des colorants basiques parmi lesquels on peut citer plus particulièrement les colorants connus dans le Color Index, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP 714954 et dont le contenu fait partie intégrante de la présente invention.

Parmi les colorants directs additionnels benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène, et
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule (II) suivante : 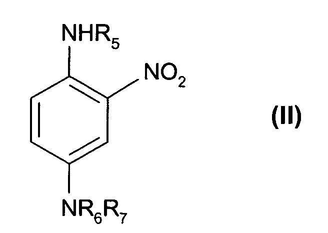
dans laquelle :
- R₆ représente un radical alkyle en C₁-C₄, un radical β-hydroxyéthyle ou β-hydroxypropyle ou γ-hydroxypropyle ;
- R₅ et R₇, identiques ou différents, représentent un radical β-hydroxyéthyle, -β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français FR 2 692 572.

Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

Toujours dans le cas où la composition est mise en oeuvre pour la coloration avec effet d'éclaircissement, de fibres kératiniques, comme les cheveux, celle-ci peut en outre comprendre au moins une base d'oxydation.

La base d'oxydation peut être choisie parmi les bases d'oxydation classiquement utilisées pour les colorations d'oxydation, comme par exemple les paraphénylènediamines, les bis-phénylalkylènediamines, les para-aminophénols, les orthoaminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) para-phénylène diamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy para-phénylènediamine, la N-(β-méthoxyéthyl) paraphénylènediamine et la 4'aminophényl 1-(3-hydroxy) pyrrolidine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylamino phényl) tétraméthylènediamine, la N,N'-bis-(éthyl) N,N'-bis-(4'-amino, 3'-méthyl phényl) éthylènediamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxy méthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut citer par exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut notamment citer les dérivés pyridiniques, les dérivés pyrimidiniques et les dérivés pyrazoliques et leurs sels d'addition avec un acide ou avec un agent alcalin.

La ou les bases d'oxydation, si elles sont présentes, représentent plus particulièrement de 0,0005 à 12 % en poids du poids total de la composition, et de préférence de 0,005 à 6 % en poids par rapport à la même référence.

Lorsqu'elle est destinée à la coloration d'oxydation de fibres kératiniques, telles que les cheveux, la composition peut également comprendre, au moins un coupleur de façon à modifier ou à enrichir en reflets les nuances obtenues en mettant en oeuvre le composé fluorescent, l'azurant optique et la base d'oxydation.

Les coupleurs utilisables peuvent être choisis parmi les coupleurs utilisés de façon classique dans ce domaine et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent par exemple de 0,0001 à 10 % en poids du poids total de la composition et plus spécialement de 0,005 à 5 % en poids par rapport à la même référence.

D'une manière générale, les sels d'addition avec un acide utilisables dans le cadre des compositions de l'invention (bases d'oxydation et coupleurs) sont notamment choisis parmi les chlorhydrates, les bromhydrates, les sulfates, les citrates, les succinates, les tartrates, les tosylates, les benzènesulfonates, les lactates et les acétates.
Les sels d'addition avec un agent alcalin utilisables dans le cadre de l'invention (bases d'oxydation et coupleurs) sont notamment choisis parmi les sels d'addition avec les métaux alcalins ou alcalino-terreux, avec l'ammoniaque, avec les amines organiques dont les alcanolamines et les composés de formule (I).

Lorsque la composition selon le procédé de l'invention est destinée à colorer les fibres kératiniques comme notamment les cheveux, elle peut renfermer au moins un agent oxydant choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

S'il est présent, la teneur en agent oxydant représente 0,001 et 10% en poids par rapport au poids de la composition tinctoriale prête à l'emploi.

La composition mise en oeuvre dans le procédé selon l'invention peut également comprendre divers adjuvants utilisés classiquement dans ce type de compositions, tels que des agents tensioactifs anioniques, cationiques, non ioniques, ou leurs mélanges, des polymères anioniques, cationiques, non ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des cations, des polymères cationiques ou amphotères, des silicones volatiles ou non volatiles, modifiées ou non modifiées, des agents filmogènes, des céramides, des agents conservateurs, des agents stabilisants, des agents opacifiants.

Parmi les agents épaississants, on préfère plus particulièrement utiliser les systèmes épaississants à base de polymères associatifs bien connus de l'homme de l'art et notamment de nature non ionique, anionique, cationique ou amphotère.

Bien entendu, l'homme de l'art veillera à choisir ce ou ces éventuels composés complémentaires de manière telle que les propriétés avantageuses attachées intrinsèquement à la composition ne soient pas, ou substantiellement pas, altérées par la ou les adjonctions envisagées.

La composition cosmétique mise en oeuvre pour colorer des fibres kératiniques, comme les cheveux, peut se présenter sous des formes diverses, telles que des lotions, des shampooings, des crèmes, des gels, des pâtes, ou sous toute autre forme appropriée.

Selon une variante avantageuse de l'invention, la composition selon le procédé de l'invention se trouve sous la forme d'un shampooing colorant et éclaircissant comprenant dans un milieu aqueux cosmétiquement acceptable, au moins un composé fluorescent et au moins un azurant optique tels que définis ci- dessus, et au moins un agent tensioactif.

De préférence, le composé fluorescent et l'azurant optique se trouvent sous une forme soluble dans le milieu.

Le ou les agents tensioactifs présents dans le shampooing peuvent être anioniques, cationiques, amphotères, ou non ioniques.
Parmi les agents tensioactifs non ioniques préférés on peut citer les alkylpolyglucosides.

Dans ces shampooings, les agents tensioactifs sont présents dans une proportion allant d'environ 4 à 30 % et de préférence d'environ 8 à 20% en poids par rapport au poids total de la composition de shampooing.

Selon une deuxième variante de l'invention, le procédé consiste à appliquer une composition sur des fibres kératiniques, notamment des cheveux, dans le but d'effectuer une coloration temporaire ou fugace avec effet d'éclaircissement, de ces fibres kératiniques, éliminable au premier shampooing ou démaquillage. Cette composition comprend de préférence au moins un composé fluorescent et au moins un azurant optique, dont au moins l'un d'entre eux et de préférence les deux, se trouvent sous une forme insoluble dans le milieu de la composition.

Dans le cas de cette variante, outre le composé fluorescent et l'azurant optique, la composition peut comprendre au moins un pigment non fluorescent.
Les pigments non fluorescents sont habituellement choisis parmi les pigments organiques ou minéraux, cosmétiquement ou dermatologiquement acceptables.
Ils peuvent se présenter sous forme de poudre ou de pâte pigmentaire.

On pourra se reporter notamment à la demande de brevet EP 808 150 pour ce qui concerne la liste des pigments utilisables.

Si les pigments non fluorescents sont présents dans la composition, leur teneur est de préférence telle qu'elle ne masque pas l'effet de fluorescence apportée par le composé fluorescent et par l'azurant optique.
A titre purement indicatif, la teneur en pigment non fluorescent, s'il est présent, est comprise entre 0,01 et 10 % en poids par rapport au poids de la composition, et de manière encore plus avantageuse, entre 0,05 et 3 % en poids par rapport à la même référence.

Le pH des compositions mises en oeuvre dans le cadre de cette deuxième variante, est plus particulièrement compris entre 6 et 8 et préférentiellement entre 6 et 7,5.

De telles compositions peuvent comprendre de plus au moins un polymère filmogène, se trouvant sous une forme soluble ou dispersée dans le milieu cosmétiquement acceptable de la composition.

Pour améliorer, si nécessaire, les propriétés du film formé, la composition peut aussi comprendre au moins un plastifiant.

Ces compositions peuvent de même contenir divers adjuvants habituellement utilisés comme par exemple les silicones insolubles ou solubles, volatiles ou non ; des protéines quaternisées ou non ; des filtres solaires ; des agents tensioactifs ; des agents antimousse ; des hydratants ; des humectants ; des émollients ; des huiles végétales ou synthétiques ; des agents conservateurs, des agents séquestrants ; des agents antioxydants ; des parfums ; des agents alcalinisants ou acidifiants, des agents de mise en suspension des pigments ; des agents épaississants.

Les compositions utilisées dans cette deuxième variante peuvent se présenter sous des formes diverses telles des liquides plus ou moins épaissis, des crèmes, des gels.
Plus spécialement, la composition peut se trouver sous la forme de mascara pour les cils ou de mascara capillaire, à appliquer notamment au pinceau ou au peigne.

Selon une troisième variante de l'invention, le procédé consiste à appliquer sur la peau dans le but de la teinter en l'éclaircissant, une composition comprenant au moins un composé fluorescent et au moins un azurant optique.

Selon cette variante, au moins le composé fluorescent se trouve sous une forme insoluble dans le milieu de la composition.

Dans le cas de cette variante, la composition comprend en général une phase grasse, dont une fraction n'est pas volatile (en d'autres termes ne s'évapore pas entre 15 et 25°C). Cette phase grasse peut constituer la phase continue ou la phase dispersée de la composition.

Ladite fraction non volatile peut être choisie parmi les huiles non volatiles, les cires, les gommes, les résines et/ou les corps gras pâteux d'origine animale, végétale, minérale ou synthétique, et/ou leurs mélanges.

Plus particulièrement, la fraction non volatile représente 1 à 85%, de préférence encore de 1 % à 30%, en poids, par rapport au poids total de la composition.

Les compositions selon cette variante de l'invention peuvent également comprendre au moins une charge particulière dite "soft-focus". Par charge, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. De plus, une charge "soft-focus" est une charge qui en plus donne de la transparence au teint et un effet flou. Cet effet soft-focus est lié à la réflectance spectrale de la charge.
Elles peuvent ainsi être choisies parmi la silice (par exemple micro-billes de silice SB-700 ou SB-150 de Miyoshi), le talc (par exemple Talc P3 de Nippon Talc), les composites silice / TiO₂ ou silice / oxyde de zinc, la poudre de polyéthylène, la poudre d'amidon, la poudre de nylon (par exemple Orgasol 2002 Extra D Nat Cos d'Atochem), les poudres de copolymères styrène/acrylique et/ou leurs mélanges.

De préférence, ces charges ont une taille moyenne des particules inférieure ou égale à 15 µm, de préférence, inférieure ou égale à 3 µm. De manière préférée, ces charges sont non sphériques.

Plus particulièrement, si elle est présente, la teneur en charge est comprise entre 0,1 à 20%, de préférence encore de 8% à 15%, en poids par rapport au poids total de la composition.

La composition selon cette variante peut de plus comprendre des additifs classiques dans le domaines, comme des filtres UV organiques hydrophiles ou liphophiles ou des filtres minéraux.
Leur teneur est habituellement comprise entre 0,1 et 20 %en poids par rapport au poids de la composition.

Par ailleurs, peuvent être présents dans cette composition, au moins un agent hydratant, comme par exemple l'urée ou ses dérivés, les polyols, comme par exemple la glycérine, le sorbitol ; des vésicules lipidiques émulsionnées notamment au moyen d'un tensioactif non ionique dans la composition, comme des protéines, des tocophérols, des acides aminés, l'allantoïne, etc.

Le pH de ce type de compositions est en général compris entre 6,5 et 7,5.

La composition selon cette variante peut se trouver notamment sous la forme d'une crème, d'un gel, d'un lait. Par exemple, la composition est un fond de teint.

Comme indiqué auparavant, les compositions mises en oeuvre dans le cadre des trois variantes et dont la nature des constituants et leurs proportions viennent d'être décrits, sont destinées à être appliquées sur des matières kératiniques.

Conformément à un premier mode de réalisation, la composition est appliquée sans rinçage puis on évapore ou on laisse évaporer le milieu.
Cette méthode est appropriée dans le cas où la matière kératinique est la peau ou si la composition est destinée à être appliquée sur des fibres kératiniques afin de teinter celles-ci de manière temporaire.

Selon un deuxième mode de l'invention, le procédé selon l'invention consiste à mettre en oeuvre les étapes suivantes :
- on applique la composition sur les matières kératiniques pendant une durée suffisante pour développer la coloration et l'éclaircissement désirés,
- on rince les matières kératiniques,
- on lave éventuellement lesdites matières avec une composition nettoyante et on les rince,
- on sèche lesdites matières kératiniques.
Cette méthode est appropriée lorsque les matières kératiniques traitées sont des fibres comme les cheveux, la moustache, la barbe, les sourcils.

Il est précisé que la composition nettoyante à laquelle il est fait référence peut être plus particulièrement un shampooing.

Le temps nécessaire au développement de la coloration et à l'obtention de l'effet éclaircissant sur les fibres kératiniques est en général d'environ 5 à 60 minutes et plus particulièrement d'environ 5 à 40 minutes.

En outre, la température nécessaire au développement de la coloration et à l'obtention de l'effet éclaircissant sur les fibres kératiniques est généralement comprise entre la température ambiante (15 à 25°C) et 80°C et plus particulièrement entre 15 et 40°C.

Toujours dans le cas de ce deuxième mode de réalisation, et plus spécialement dans celui de la coloration directe ou d'oxydation, le procédé selon l'invention comporte une étape préliminaire consistant à stocker sous forme séparée, d'une part, une composition comprenant, dans un milieu approprié pour la teinture, au moins un composé fluorescent et au moins un azurant optique, et éventuellement au moins un colorant direct additionnel et/ou au moins une base d'oxydation et/ou au moins un coupleur, et d'autre part, une composition renfermant, dans un milieu approprié pour la teinture, au moins un agent oxydant ; puis à procéder à leur mélange au moment de l'emploi avant d'appliquer ce mélange sur les fibres kératiniques pendant un temps suffisant pour développer la coloration et l'éclaircissement désirés, après quoi on rince les fibres kératiniques ; on lave éventuellement lesdites fibres kératiniques avec une composition nettoyante et on rince ; puis on sèche les fibres kératiniques.

Un autre objet de l'invention est constitué par une composition comprenant, dans un milieu cosmétiquement acceptable, au moins un composé fluorescent soluble dans le milieu et au moins un azurant optique soluble dans le milieu.

Plus particulièrement, le composé fluorescent est choisi parmi les composés solubles dans le milieu de la composition à au moins 1 g/l et de préférence à au moins 5 g/l, à une température comprise entre 15 et 25°C.
En ce qui concerne l'azurant optique, ce dernier est choisi parmi les composés qui sont solubles dans le milieu de la composition à au moins 0,1 g/l et de préférence à au moins 0,5 g/l, à une température comprise entre 15 et 25°C.

Les listes de composés fluorescents et azurant optiques solubles données auparavant restent valables et l'on pourra s'y référer.

Les quantités de composé(s) fluorescent(s) et d'azurant(s) optique(s) présents dans la composition, sont plus particulièrement telles qu'après application sur la matière kératinique, dont la hauteur de ton est d'au plus 6, de préférence d'au plus 4, la composition donne une réflectance, mesurée entre 550 et 700 nm, supérieure à la réflectance de la matière kératinique non traitée.

Plus spécialement, la teneur en composé(s) fluorescent(s) représente 0,05 à 20 % en poids par rapport au poids de la composition, plus particulièrement 0,05 à 10% en poids par rapport à la même référence, de préférence 0,1 à 5% en poids par rapport à la même référence.

De plus, la teneur en azurant optique représente plus particulièrement 0,05 à 10% en poids par rapport au poids total de la composition, de préférence 0,1 à 5% en poids par rapport à la même référence.

Le milieu cosmétiquement acceptable est généralement constitué par de l'eau ou par un mélange d'eau et d'au moins un solvant organique.

A titre de solvant organique convenable, on peut citer entre autres, les alcanols, linéaires ou ramifiés, comprenant 1 à 4 atomes de carbone ; les polyols et éthers de polyols ; les alcools aromatiques, ces composés étant seuls ou en mélanges.

Habituellement, les solvants, s'ils sont présents, représentent 1 à 40 % en poids environ par rapport au poids total de la composition, et de manière plus avantageuse de 5 à 30 % en poids environ par rapport à la même référence.

Le pH de la composition mise en oeuvre dans l'invention est généralement compris entre 3 et 12 environ, et de préférence entre 5 et 11 environ.

Il peut être ajusté à la valeur désirée au moyen d'agents acidifiants ou alcalinisants habituellement utilisés dont les listes ont été indiquées auparavant.

La composition selon l'invention peut en outre comprendre au moins un colorant direct additionnel. Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

La composition peut de même comprendre au moins une base d'oxydation.
Si elle est utilisée, elle représente en général de 0,0005 à 12 % en poids du poids total de la composition, et de préférence de 0,005 à 6 % en poids par rapport à la même référence.

Par ailleurs, la composition peut comprendre un ou plusieurs coupleurs, de façon à modifier ou à enrichir en reflets les nuances obtenues en mettant en oeuvre les composés fluorescents, azurants optiques et la ou les bases d'oxydation.
Lorsqu'ils sont présents, le ou les coupleurs représentent par exemple de 0,0001 à 10 % en poids du poids total de la composition et plus spécialement de 0,005 à 5 % en poids par rapport à la même référence.

Conformément à un mode de réalisation de l'invention, la composition comprend au moins un agent oxydant.
S'il est présent, la teneur en agent oxydant représente 0,001 à 10% en poids par rapport au poids de la composition tinctoriale.

La composition mis en oeuvre dans le procédé selon l'invention peut également comprendre divers adjuvants utilisés classiquement dans ce type de compositions.

Pour ce qui concerne la nature des colorants directs, bases d'oxydation, coupleurs et autres adjuvants de la composition, on pourra se reporter aux listes données dans le cadre de la description des compositions mise en oeuvre dans le procédé selon l'invention.

La composition cosmétique mise en oeuvre pour colorer les fibres kératiniques peut se présenter sous des formes diverses, telles que des lotions, des shampooings, des crèmes, des gels, des pâtes, ou sous toute autre forme appropriée.

Selon une variante avantageuse de l'invention, la composition se trouve sous la forme d'un shampooing colorant et éclaircissant comprenant dans un milieu aqueux cosmétiquement acceptable, au moins un composé fluorescent et au moins un azurant optique tels que définis ci-dessus, et au moins un agent tensioactif.

Le ou les agents tensioactifs présents dans le shampooing peuvent être anioniques, cationiques, amphotères ou non ioniques.
Parmi les agents tensioactifs non ioniques préférés, on peut citer les alkylpolyglucosides.

Dans ces shampooings, les agents tensioactifs sont présents dans une proportion allant d'environ 4 à 30 % et de préférence d'environ 8 à 20% en poids par rapport au poids total de la composition de shampooing.

Un autre objet de l'invention est un dispositif à plusieurs compartiments, comprenant au moins un compartiment renfermant une composition comprenant au moins un composé fluorescent et au moins un azurant optique et éventuellement au moins un colorant direct additionnel et/ou au moins une base d'oxydation et/ou au moins un coupleur, dans un milieu cosmétiquement acceptable, et au moins un autre compartiment renfermant une composition comprenant au moins un agent oxydant.

Selon un mode de réalisation particulier de dispositif de l'invention, la composition comprend au moins un composé fluorescent et au moins un azurant optique solubles dans le milieu telle qu'elle vient d'être décrite.

Le dispositif à plusieurs compartiments peut être équipé d'un moyen permettant de délivrer sur les cheveux le mélange souhaité, tel que les dispositifs décrits dans le brevet français FR 2 586 913.

Des exemples concrets mais non limitatifs de l'invention vont maintenant être présentés.

### EXEMPLES

On a préparé les trois compositions de teinture directe suivantes (teneurs exprimées en grammes) :

| Compositions | 1 comparatif | 2 invention | 3 invention |
|---|---|---|---|
| Colorant fluorescent (a) | 0,5 | 0,5 | 0,5 |
| Azurant optique Diéthylaminométhyl coumarine | - | 0,5 | - |
| Azurant optique 4-méthyl 7-diéthyl coumarine | - | - | 0,5 |
| Hydroxyéthylcellulose | 1,6 | 1,6 | 1,6 |
| Alkyl (C₈/C₁₀ - 50/50) polyglucoside en solution aqueuse à 60% tamponnée | 6 MA* | 6 MA* | 6 MA* |
| Alcool benzylique | 8 | 8 | 8 |
| Polyéthylène glycol | 12 | 12 | 12 |
| Mélange de p-hydroxybenzoates de méthyle, butyle, éthyle, propyle, isobutyle | 0,12 | 0,12 | 0,12 |
| Eau déminéralisée .........q.s.p. | 100 | 100 | 100 |

| | | | |
|---|---|---|---|
| MA* désigne Matière Active (a) le colorant fluorescent est le Photosensitiving Dye NK-557 commercialisé par la société UBICHEM. | | | |

Les essais sont faits sur des mèches de cheveux châtains, de hauteur de ton 4.

La composition est appliquée sur des cheveux châtains à raison de 10 grammes de composition pour 1 gramme de cheveux châtains. La composition est étalée de façon à recouvrir l'ensemble des cheveux. On laisse la composition agir pendant 20 minutes à température ambiante (20 à 25°C). Les cheveux sont ensuite rincés à l'eau puis lavés avec un shampooing à base de lauryléther sulfate. Ils sont ensuite séchés.

Les performances d'éclaircissement des compositions conformes à l'invention, exprimées en fonction de la réflectance des cheveux, sont comparées à la réflectance d'une mèche de cheveux non traitées et à celle obtenue avec le produit commercial d'éclaircissement "Lumiblonde" (décoloration chimique).

La réflectance est mesurée au moyen d'un appareil spectrophotocolorimètre et après irradiation des cheveux avec une lumière visible dans la gamme de longueurs d'onde allant de 400 à 700 nanomètres.

La figure 1 représente la réflectance en fonction de la longueur d'onde, de cheveux châtains non traités, de cheveux traités avec le produit commercial "Lumiblonde", de cheveu traités avec la composition 1 (composé fluorescent seul) et avec la composition 2 (composé fluorescent et azurant optique).

La figure 2 représente la réflectance en fonction de la longueur d'onde, de cheveux châtains non traités, de cheveux traités avec le produit commercial "Lumiblonde", de cheveu traités avec la composition 1 (composé fluorescent seul) et avec la composition 3 (composé fluorescent et azurant optique).

On constate tout d'abord que la réflectance d'une mèche de cheveux traitée avec une composition selon l'invention est supérieure à celle des cheveux non traités. Les mèches traitées apparaissent donc plus claires.
Il est à noter que la réflectance d'une mèche de cheveux traitées avec une composition comprenant 0,5 g d'un azurant optique est voisine de celle d'une mèche de cheveux non traités. Aucun effet d'éclaircissement n'est observé dans ce cas.

De plus, l'association du composé fluorescent et de l'azurant optique donne des pourcentages de réflectance supérieurs à ceux obtenus avec le composé fluorescent seul et cela sur une gamme de longueur d'onde plus étendue que celle obtenue avec le composé fluorescent seul.

Ainsi, la composition comprenant une association du composé fluorescent et de l'azurant optique permet en plus d'obtenir un effet d'éclaircissement plus marqué, plus naturel et esthétique que celui obtenu avec le composé fluorescent seul.

## Revendications

1. Procédé pour teinter ou colorer avec un effet éclaircissant des matières kératiniques humaines, consistant à appliquer sur lesdites matières une composition comprenant, dans un milieu cosmétiquement acceptable, au moins un composé fluorescent et au moins un azurant optique.

2. Procédé selon la revendication précédente, **caractérisé en ce que** l'on applique la composition sur les matières kératiniques sans rinçage puis on évapore ou on laisse évaporer ledit milieu.

3. Procédé selon la revendication 1, **caractérisé en ce que** l'on met en oeuvre les étapes suivantes :
- on applique la composition sur les matières kératiniques pendant une durée suffisante pour développer la coloration et l'éclaircissement désirés,
- on rince les matières kératiniques,
- on lave éventuellement lesdites matières avec une composition nettoyante et on les rince,
- on sèche lesdites matières kératiniques.

4. Procédé selon l'une des revendications précédentes, **caractérisé en ce que** le composé fluorescent absorbe la lumière dans la partie visible du spectre et éventuellement dans la zone de l'ultraviolet, et ré-émet une lumière fluorescente de plus grande longueur d'onde dans le spectre du visible, comprise entre 500 et 700 nanomètres.

5. Procédé selon l'une des revendications précédentes, **caractérisé en ce que** l'azurant optique absorbe la lumière dans la partie ultraviolette du spectre, et ré-émet une lumière fluorescente dans le spectre du visible, comprise entre 400 et moins de 525 nanomètres.

6. Procédé selon l'une des revendications précédentes, **caractérisé en ce que** la quantité de composé fluorescent et la quantité d'azurant optique sont telles qu'après application sur les matières kératiniques, la composition donne une réflectance, mesurée entre 550 et 700 nm, qui est supérieure à la réflectance desdites matières kératiniques non traitées.

7. Procédé selon l'une des revendications précédentes, **caractérisé en ce que** la teneur en composé fluorescent représente 0,05 à 20% en poids par rapport au poids total de la composition, plus particulièrement 0,05 à 10% en poids par rapport à la même référence, de préférence 0,1 à 5% en poids par rapport à la même référence.

8. Procédé selon l'une des revendications précédentes, **caractérisé en ce que** la teneur en azurant optique représente 0,05 à 10% en poids par rapport au poids total de la composition, de préférence 0,1 à 5% en poids par rapport à la même référence.

9. Procédé selon l'une des revendications précédentes, **caractérisé en ce que** le milieu cosmétiquement acceptable comprend de l'eau et éventuellement un solvant organique.

10. Procédé selon l'une des revendications précédentes, **caractérisé en ce que** le pH de la composition est compris entre 3 et 12, de préférence entre 5 et 11.

11. Procédé selon l'une des revendications 3 à 10, **caractérisé en ce que** la composition comprend au moins un colorant direct additionnel de nature non ionique, cationique ou anionique.

12. Procédé selon la revendication précédente, **caractérisé en ce que** le colorant direct additionnel est choisi parmi les colorants benzéniques nitrés.

13. Procédé selon la revendication 12, **caractérisé en ce que** le colorant direct additionnel est choisi parmi les colorants azoïques, les colorants anthraquinoniques, naphtoquinoniques ou benzoquinoniques, les colorants indigoïdes, ou les colorants dérivés du triarylméthane.

14. Procédé selon l'une des revendications 11 à 13, **caractérisé en ce que** la teneur totale en colorants directs additionnels représente de 0,0005 à 12 % en poids par rapport au poids total de la composition, de préférence de 0,005 à 6 % en poids par rapport à la même référence.

15. Procédé selon l'une des revendications 3 à 14, **caractérisé en ce que** la composition comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

16. Procédé selon la revendication précédente, **caractérisé en ce que** la teneur totale en base d'oxydation est comprise entre 0,0005 et 12 % en poids par rapport au poids total de la composition, de préférence entre 0,005 et 6 % en poids par rapport à la même référence.

17. Procédé selon l'une des revendications 15 ou 16, **caractérisé en ce que** la composition comprend au moins un coupleur choisi parmi les métaphénylènediamines, les méta-aminophénols, les métadiphénols et les coupleurs hétérocycliques ou leurs sels d'addition avec un acide ou avec un agent alcalin.

18. Procédé selon la revendication précédente, **caractérisé en ce que** la teneur totale en coupleur est comprise entre 0,0001 et 10 % en poids par rapport au poids total de la composition, de préférence de 0,005 à 5 % en poids par rapport à la même référence.

19. Procédé selon l'une des revendications 3 à 18, **caractérisé en ce que** la composition comprend au moins un agent oxydant.

20. Procédé selon la revendication précédente, **caractérisé en ce que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou quatre électrons.

21. Procédé selon l'une des revendications précédentes, **caractérisé en ce que** la composition comprend au moins un tensioactif anionique, cationique,amphotère ou non ionique.

22. Procédé selon l'une des revendications 3 à 21, **caractérisé en ce qu'**il comporte une étape préliminaire consistant à stocker sous forme séparée, d'une part, une composition comprenant, dans un milieu approprié pour la teinture, au moins un composé fluorescent et au moins un azurant optique, et éventuellement au moins un colorant direct additionnel et/ou au moins une base d'oxydation et/ou au moins un coupleur, d'autre part, une composition renfermant, dans un milieu approprié pour la teinture, au moins un agent oxydant ; puis à procéder à leur mélange au moment de l'emploi avant d'appliquer ce mélange sur les cheveux pendant un temps suffisant pour développer la coloration désirée, après quoi on rince les cheveux ; on lave éventuellement les cheveux avec une composition nettoyante et on rince ; puis on sèche les cheveux.

23. Procédé selon l'une des revendications 3 à 21, **caractérisé en ce que** la composition se trouve sous la forme d'un shampooing colorant.

24. Procédé selon l'une des revendications 2, 4 à 10, **caractérisé en ce que** la composition comprend au moins un pigment non fluorescent.

25. Procédé selon la revendication précédente, **caractérisé en ce que** la teneur en pigment est comprise entre 0,01 et 10 % en poids de la composition, de préférence entre 0,05 et 3 % en poids de la composition.

26. Procédé selon l'une des revendications 24 ou 25, **caractérisé en ce que** le pH de la composition est compris entre 6,5 et 7,5.

27. Procédé selon l'une des revendications 24 à 26, **caractérisé en ce que** la composition est sous forme d'un mascara pour les cils ou d'un mascara capillaire.

28. Procédé selon l'une des revendications 1 ou 2, **caractérisé en ce que** les matières kératiniques désignent la peau ou des fibres kératiniques.

29. Procédé selon la revendication précédente, **caractérisé en ce que** la peau présente une luminance L* dans le système C.I.E. L*a*b* inférieure ou égale à 55.

30. Procédé selon l'une des revendications 1 ou 3, **caractérisé en ce que** les matières kératiniques sont des fibres kératiniques.

31. Procédé selon la revendication 28 ou 30, **caractérisée en ce que** les fibres kératiniques présentent une hauteur de ton inférieure ou égale à 6 et de préférence inférieure ou égale à 4.

32. Composition comprenant, dans un milieu cosmétiquement acceptable, au moins un composé fluorescent soluble dans le milieu et au moins un azurant optique soluble dans le milieu.

33. Composition selon la revendication précédente, **caractérisée en ce que** le composé fluorescent absorbe la lumière dans la partie visible du spectre et éventuellement dans la zone de l'ultraviolet, et ré-émet une lumière fluorescente de plus grande longueur d'onde dans le spectre du visible, comprise entre 500 et 700 nm.

34. Composition selon la revendication précédente, **caractérisée en ce que** la teneur en composé fluorescent représente 0,05 à 20 % en poids par rapport au poids de la composition, plus particulièrement 0,05 à 10% en poids par rapport à la même référence, de préférence 0,1 à 5% en poids par rapport à la même référence.

35. Composition selon l'une des revendications 32 à 34, **caractérisée en ce que** l'azurant optique absorbe la lumière dans la partie ultraviolette du spectre, et ré-émet une lumière fluorescente dans le spectre du visible, comprise entre de 400 à 525 nm.

36. Composition selon la revendication précédente, **caractérisée en ce que** la teneur en azurant optique représente 0,05 à 10% en poids par rapport au poids total de la composition, de préférence 0,1 à 5% en poids par rapport à la même référence.

37. Composition selon l'une des revendications 32 à 36, **caractérisée en ce que** le milieu cosmétiquement acceptable comprend de l'eau et éventuellement un solvant organique.

38. Composition selon l'une des revendications 32 à 37, **caractérisée en ce que** le pH de la composition est compris entre 3 et 12, de préférence entre 5 et 11.

39. Composition selon l'une des revendications 32 à 38, **caractérisée en ce que** la composition comprend au moins un colorant direct additionnel de nature non ionique, cationique ou anionique.

40. Composition selon la revendication précédente, **caractérisée en ce que** le colorant direct additionnel est choisi parmi les colorants benzéniques nitrés.

41. Composition selon la revendication 39, **caractérisée en ce que** le colorant direct additionnel est choisi parmi les colorants azoïques, les colorants anthraquinoniques, naphtoquinoniques ou benzoquinoniques, les colorants indigoïdes, ou les colorants dérivés du triarylméthane.

42. Composition selon l'une des revendications 39 à 41, **caractérisée en ce que** la teneur totale en colorants directs additionnels représente de 0,0005 à 12 % en poids par rapport au poids total de la composition, de préférence de 0,005 à 6 % en poids par rapport à la même référence.

43. Composition selon l'une des revendications 32 à 42, **caractérisée en ce que** la composition comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

44. Composition selon la revendication précédente, **caractérisée en ce que** la teneur totale en base d'oxydation est comprise entre 0,0005 et 12 % en poids par rapport au poids total de la composition, de préférence entre 0,005 et 6 % en poids par rapport à la même référence.

45. Composition selon l'une des revendications 43 ou 44, **caractérisée en ce que** la composition comprend au moins un coupleur choisi parmi les métaphénylènediamines, les méta-aminophénols, les métadiphénols et les coupleurs hétérocycliques ou leurs sels d'addition avec un acide ou avec un agent alcalin.

46. Composition selon la revendication précédente, **caractérisée en ce que** la teneur totale en coupleur est comprise entre 0,0001 et 10 % en poids par rapport au poids total de la composition, de préférence de 0,005 à 5 % en poids par rapport à la même référence.

47. Composition selon l'une des revendications 32 à 46, **caractérisée en ce que** la composition comprend au moins un agent oxydant.

48. Composition selon la revendication précédente, **caractérisée en ce que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou quatre électrons.

49. Composition selon l'une des revendications 32 à 48, **caractérisée en ce que** la composition comprend au moins un tensioactif anionique,amphotère, cationique ou non ionique.

50. Composition selon l'une des revendications 32 à 49, **caractérisée en ce qu'**elle se trouve sous la forme d'une composition sous la forme d'une crème, d'une pâte, d'un gel.

51. Composition selon l'une des revendications 32 à 42 et 49, **caractérisée en ce qu'**elle se trouve sous la forme d'un shampooing colorant.

52. Dispositif à plusieurs compartiments, comprenant au moins un compartiment renfermant une composition comprenant au moins un composé fluorescent et au moins un azurant optique et éventuellement au moins un colorant direct additionnel et/ou au moins une base d'oxydation et/ou au moins un coupleur, dans un milieu cosmétiquement acceptable, et au moins un autre compartiment renfermant une composition comprenant au moins un agent oxydant.

53. Dispositif selon la revendication précédente, **caractérisé en ce que** le ou les composés fluorescents et le ou les azurants optiques sont solubles dans le milieu.
